# EUROPEAN PATENT APPLICATION

(11) **EP 3 361 256 A1**
(43) Date of publication of application: **15.08.2018**
(21) Application number: 17382054.9
(22) Date of filing: 08.02.2017
(51) Int. Cl.: G01N 33/574

(54) **IN VITRO METHOD FOR THE DIAGNOSIS OF LUNG CANCER**

(71) Applicant: Fundación para la Investigación Médica Aplicada, 31008 Pamplona (ES); Advanced Marker Discovery, S.L., 47004 Valladolid (ES)
(72) Inventor: AGORRETA ARRAZUBI, Jackeline, 31008 Pamplona (ES); AJONA MARTÍNEZ-POLO, Daniel, 31008 Pamplona (ES); MONTUENGA BADÍA, Luis, 31008 Pamplona (ES); PAJARES VILLANDIEGO, María Josefa, 31008 Pamplona (ES); PÍO OSÉS, Rubén, 31008 Pamplona (ES)
(74) Representative: Hoffmann Eitle

(57) **Abstract**

*In vitro* method for the diagnosis of lung cancer. The present invention is generally related to diagnostic assays. In particular, the present invention refers to the use of at least C4c fragment as diagnostic and prognostic lung cancer marker. C4c fragment can also be useful to estimate lung cancer risk and to decide whether a medical regimen has to be initiated and to determine whether the medical regimen initiated is efficient.

## Description

### FIELD OF THE INVENTION

The present invention is generally related to diagnostic and prognostic assays. In particular, the present invention refers to the use of at least C4c fragment as diagnostic and prognostic lung cancer marker. C4c fragment can also be useful to estimate lung cancer risk and to decide whether a medical regimen has to be initiated and to determine whether the medical regimen initiated is efficient.

### STATE OF THE ART

Lung cancer is the leading cause of cancer-related death worldwide. Lung cancer comprises two main histological subtypes: small cell lung cancer (SCLC) and non-small cell lung cancer (NSCLC). The latter accounts for 80-85% of all cases and includes the two most frequent lung cancer types: adenocarcinomas and squamous cell carcinomas. Irrespective of the histology, most lung cancer patients are diagnosed at advanced stages, when the disease is almost incurable.

Several experimental observations suggest that complement, as part of the immune surveillance system, is activated in patients with neoplastic diseases, including lung cancer. The complement system is a central part of the innate immune response that has developed as a first defense against pathogens or unwanted host elements. This system consists of more than 30 soluble proteins, surface regulatory factors and receptors that work together to accomplish a variety of activities that range from cell cytotoxicity to the regulation of adaptive immunity and tissue homeostasis. Complement can be activated through three major pathways, the classical, the alternative and the lectin pathways, which converge in the cleavage of C3 into C3b. C3b deposition leads to the formation of C3 convertases that amplify complement response, and eventually promote the formation of the C5 convertase and the assembly of the membrane attack complex (MAC). The three pathways of complement activation differ from each other in the mechanism of target recognition and initiation. The classical pathway of complement is normally initiated by the binding of C1q to Fc regions of antigen-bound immunoglobulins (IgG or IgM). C1q together with C1r and C1s, two serine protease proenzymes, constitute the C1 complex, the first component of the classical pathway. The C1 complex cleaves C4 and C2 to yield the classical pathway C3 convertase (C4b2a), which is able to activate C3. The alternative pathway is initiated by low-level activation of C3 to C3b by spontaneously hydrolyzed C3 and activated factor B. C3b can attach to the target cell membrane, and bind to factor B that is cleaved by factor D to form the alternative pathway C3 convertase (C3bBb). The lectin pathway is activated following the recognition and binding of mannose-binding lectin (MBL) to repetitive carbohydrate patterns containing mannose and N-acetylglucosamine residues on pathogen surfaces. MBL forms a C1-like complex with MBL-associated serine proteases (MASP). Conformational changes in MBL lead to the cleavage and activation of complement components C4 and C2, which continue activation as in the classical pathway. In all three pathways, cleavage and activation of C3 results in the deposition of C3b on the surface of the target cell, leading to the activation of the C5-C9 components and the formation of the cytolytic membrane attack complex (MAC) that binds to cell membranes, disrupts the membrane's integrity and facilitates cell lysis. Finally, along the complement cascade, C4a, C3a and C5a are released. These peptides, known as anaphylatoxins, are generated by the proteolysis of complement components C4, C3 and C5 and exert various biological functions important for the initiation and maintenance of inflammatory responses.

In the case of lung tumors, immunohistochemical analyses revealed the deposition of C3b in primary tumors, with an apparent lack of activation of the lytic MAC. Besides, elevated complement levels correlating with tumor size were found in lung cancer patients, and complement components C3c and C4 were significantly elevated in patients with lung cancer when compared with a control group. Many proteomic studies have also reported an elevation of complement components in the plasma of lung cancer patients. Using functional analyses, we have previously proposed that NSCLC cells activate complement more efficiently than their non-malignant counterparts. These analyses demonstrate that C1 q directly binds to lung cancer cells and activates the classical pathway in an antibody-independent manner. In agreement with this observation, C4d, a complement split product derived from the classical pathway activation, is found deposited in lung primary tumors (Ajona D, Pajares MJ, Corrales L, Perez-Gracia JL, Agorreta J, Lozano MD, Torre W, Massion PP, de-Torres JP, Jantus-Lewintre E, Camps C, Zulueta JJ, Montuenga LM, Pio R. Investigation of complement activation product c4d as a diagnostic and prognostic biomarker for lung cancer. J Natl Cancer Inst 2013; 105: 1385-1393).

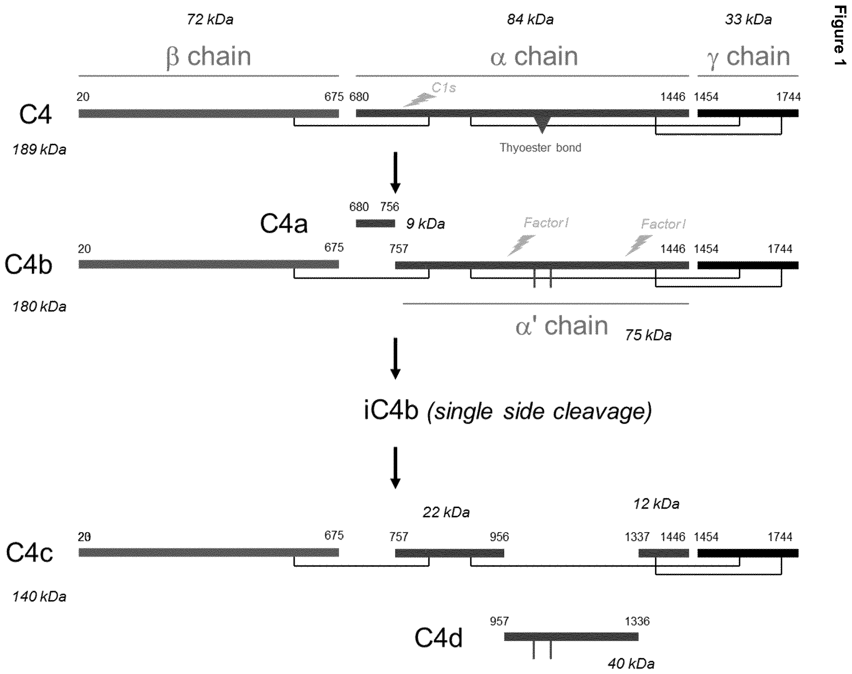

The mature form of C4 contains three chains (α, β and γ)(**Figure 1**). After complement activation, C1s cleaves a single peptide bond in the alpha chain and generates C4b. This cleavage involves a conformational change that leads to the exposure of a thioester group. This activated group can bind covalently to target cell surfaces by amide or ester bonds. Surface-bound C4b combines with C2a to form the C4b2a complex, the C3-convertase of the classical pathway. In order to protect normal host cells from bystander killing, the activation of the complement cascade is highly controlled by several regulatory proteins. Accordingly, C4b is cleaved by the regulatory protein factor I into iC4b, and finally into C4c and C4d. C4c is released to the extracellular medium after C4 fragmentation, whereas most C4d remains covalently-attached to the plasma membrane. The levels of C4d-containing fragments can be significantly elevated in samples from patients with a variety of autoimmune diseases (rheumatoid arthritis, hereditary angioedema, systemic lupus erythematosus, etc.), in which activation of the classical complement pathway is known to occur. Detection of C4d is also an established marker for antibody-mediated rejection in allograft rejection.

Based on the observation that lung cancer cells are able to activate the classical pathway and, consequently, to induce the proteolysis of C4, a new strategy was proposed to evaluate the possibility of using specific elements of this pathway as lung cancer biomarkers, provided that these elements could be detected in biological fluids. This strategy was based on the determination of C4d-containing fragments of activated C4 (a term that comprises the molecules C4b, iC4b and C4d).

Cancer markers can provide guidance for the clinical management of patients. They can be useful for prediction of an individual's risk of developing cancer, early diagnosis, diagnostic work-up of a patient suspected to have cancer, prediction of the aggressiveness of the tumor (prognostic value), guidance on the selection of the more appropriate therapy (predictive value), and monitoring of the patient's response to a specific treatment and of the potential relapse of the disease after treatment. A successful biomarker should be found in specimens obtained from relatively noninvasive procedures, and be associated with high sensitivity and specificity. One of the most adequate samples to evaluate biomarkers is blood (plasma or serum), a type of sample that can be easily and inexpensively collected by minimally invasive procedures. Although significant advances in the understanding of the molecular and genetic alterations in lung cancer have occurred, nowadays, there are not molecular markers available that can be routinely used for risk assessment, early detection, diagnosis, prognosis, or monitoring treatment response in lung cancer.

A lack of suitable techniques or biomarkers for early detection is one of the main reasons behind the dismal statistics related to lung cancer clinical outcomes. Nowadays, only 20% of patients are diagnosed in early stages (I and II), when surgical intervention is possible. This scenario may change in the future, since extensive efforts are devoted to significantly increase the percentage of these early detected cases. In this regard, low-dose circular tomography (CT)-based lung cancer screening studies have reported high rates of detection of small cancers in early stages. The National Lung Screening Trial-NLST, which included more than 50,000 participants, concluded that screening with the use of spiral CT detects lung tumors at early stages (mostly stage I) and reduces mortality from lung cancer. The U.S. Preventive Services Task Force recommends annual screening for lung cancer with low-dose CT in adults, aged 55 to 80 years, who have a 30 pack-year smoking history and currently smoke or have quit within the past 15 years. Similar recommendations are also made by numerous other institutions such as the American Lung Association, the National Comprehensive Cancer Network and the American Cancer Society. Since February 2015, Medicare beneficiaries who meet inclusion criteria are covered for lung cancer screening.

A critical aspect in CT-screening programs is the management of indeterminate pulmonary nodules. The average pulmonary nodule detection rate in randomized low dose-CT screening clinical trials is around 25%, with a large majority of them (around 96%) being benign. Therefore, an appropriate prediction of malignancy once a pulmonary nodule is detected in screened subjects would reduce the number of CT-screening rounds, unnecessary diagnostic follow-up procedures (and the associated risk of morbidity) and cost for healthcare systems. Several predictive models have been proposed enabling quantification of malignancy risk for a given nodule. These models take into account clinical and demographic factors, as well as quantitative and qualitative analyses of nodule images obtained from CT and positron emission tomography scans. However, current predictive tools to discriminate benign from malignant nodules are suboptimal, and the development of complementary molecular biomarkers may be very useful.

### DESCRIPTION OF THE INVENTION

### Brief description of the invention

The use of biomarkers in the context of lung cancer screening programs has an enormous potential. In particular, discriminating whether incidental or screening-detected pulmonary nodules are malignant or benign represents one of the most urgent clinical problems in early detection of lung cancer. Unfortunately, current predictive tools to discriminate benign from malignant nodules are suboptimal, and cancer research has not yet accomplished the goal of producing a marker that can be routinely used in the clinic.

Some studies had previously determined the presence of complement components on biological fluids from patients with lung cancer. Promising results were obtained by the determination of C4d-containing fragments of activated C4, which, as shown in **Figure 1****,** comprises the molecules C4b, iC4b and C4d. The quantification of these markers in biological fluids from lung cancer patients could be of clinical use for risk assessment, diagnosis, and monitoring of response.

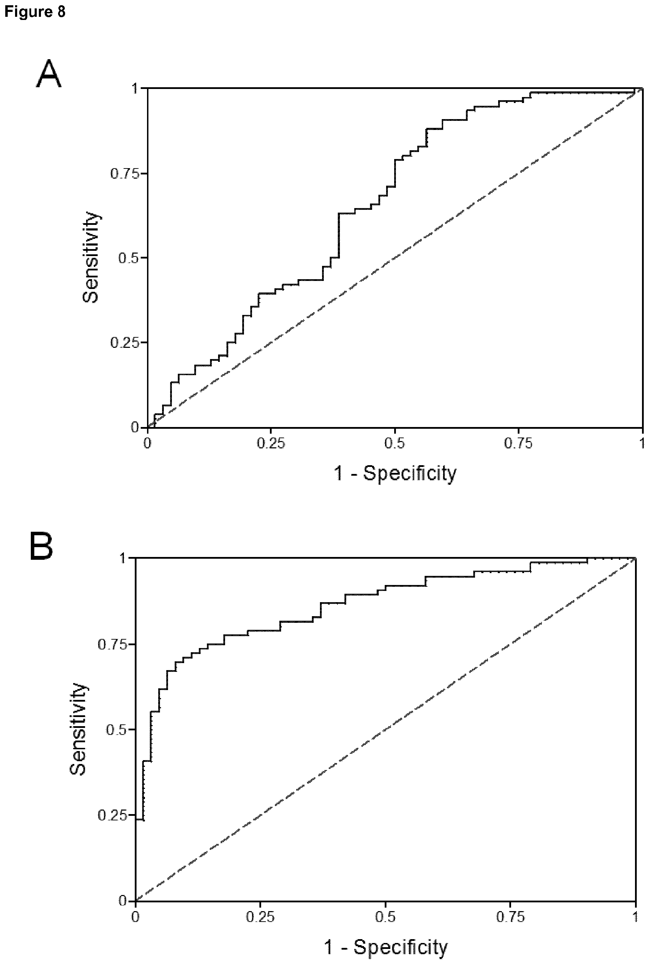

The present invention surprisingly evidences that the determination of C4c fragment (herein also abbreviated as C4c), another element produced from C4 after complement activation, provides a substantial advantage over the determination of other fragments from C4 proteolysis in the diagnosis of lung cancer. In particular, C4c fragment can be determined in a plasma sample in a meaningful manner for the diagnosis of lung cancer in a subject (see **Example 1,** in particular table 4, figure 8 and table 11). Moreover, a model defined by the combination of C4c with other protein markers, namely CYFRA 21-1 and/or C-reactive protein (CRP) and/or prolactin, yields a better diagnostic performance than the determination of C4c alone. This invention also evidences that the quantification of C4c, and its combination with the above mentioned markers, can provide information as to whether a suspicious indeterminate pulmonary nodule is malignant or not. Moreover, this information can be combined with epidemiological and clinical data to generate a diagnostic model for prediction of malignancy. This invention may improve diagnostic and screening efficacies, particularly by discriminating which of the nodules, incidentally-found or screening-detected, may need more active follow up. The application of this invention may be used to improve the clinical management of lung nodules, reducing the number of potentially harmful invasive procedures carried out to diagnose the disease.

Consequently, the first embodiment of the present invention refers to an *in vitro* method for the diagnosis of lung cancer in a subject comprising: a) determining the level of at least C4c fragment in a sample obtained from the subject; comparing the C4c fragment level determined in step (a) with a reference control level of said C4c fragment, and wherein if the C4c fragment level determined in step (a) is higher than the reference control level, it is indicative that the subject suffers from lung cancer and/or that the pulmonary nodule/s present in the subject are deemed to be malignant. In a preferred embodiment, the present invention refers to an *in vitro* method for the diagnosis of lung cancer in a subject comprising: a) determining the levels of C4c fragment and prolactin, C4c fragment and CYFRA 21-1, C4c fragment and CRP, C4c fragment and prolactin and CYFRA 21-1, C4c fragment and prolactin and CRP, or C4c fragment and CYFRA 21-1 and CRP, or C4c fragment and CYFRA 21-1 and CRP and prolactin, in a sample obtained from the subject; and comparing the levels determined in step (a) with reference control levels of said biomarkers, and wherein if the levels determined in step (a) are higher than the reference control levels, it is indicative that the subject suffers from lung cancer and/or that the pulmonary nodules present in the subject are deemed to be malignant. In a preferred embodiment of the invention lung cancer is selected from the group consisting of non-small cell lung cancer and small-cell lung carcinoma. In a preferred embodiment of the invention the sample is selected from: blood, plasma, serum, bronchoalveolar lavage fluid, sputum, biopsy and surgical specimens. A plasma sample is particularly preferred.

The second embodiment of the present invention refers to *an vitro* method for deciding or recommending a medical treatment to a subject suffering from lung cancer comprising: determining the level of at least C4c fragment in a sample obtained from the subject; and comparing the C4c fragment level determined in step (a) with a reference control level of said C4c fragment, and wherein if the C4c fragment level determined in step (a) is higher than the reference control level, a medical treatment is recommended or if the C4c fragment level determined in step (a) is not higher than the reference control level, a follow-up is then recommended. In a preferred embodiment of the invention the present invention refers to *an vitro* method for deciding or recommending a medical treatment to a subject suffering from lung cancer comprising: a) determining the levels of C4c fragment and prolactin, C4c fragment and CYFRA 21-1, C4c fragment and CRP, C4c fragment and prolactin and CYFRA 21-1, C4c fragment and prolactin and CRP, or C4c fragment and CYFRA 21-1 and CRP, or C4c fragment and CYFRA 21-1 and CRP and prolactin, in a sample obtained from the subject; and comparing the levels determined in step (a) with reference control levels of said biomarkers, wherein if the levels determined in step (a) are higher than the reference control levels, a medical treatment is recommended, or if the levels determined in step (a) are not higher than the reference control levels, a follow-up is then recommended. In a preferred embodiment of the invention, lung cancer is selected from the group consisting of non-small cell lung cancer and small-cell lung carcinoma. In a preferred embodiment of the invention the sample is selected from: blood, plasma, serum, bronchoalveolar lavage fluid, sputum, biopsy and surgical specimens. A plasma sample is particularly preferred.

The third embodiment of the present invention refers to an *in vitro* method for determining the efficacy of a medical regimen in a patient already diagnosed with lung cancer comprising: a) measuring the level of at least C4c fragment in a sample obtained from the patient prior to the administration of the medical regimen; and b) measuring the C4c fragment level of said C4c fragment in a sample from the patient once started the administration of the medical regimen; and c) comparing the C4c fragment levels measured in steps (a) and (b), in such a way that if the C4c fragment level measured in step (b) is lower than the C4c fragment level measured in step (a), it is indicative that the medical regimen is effective in the treatment of lung cancer. In a preferred embodiment the present invention refers to *an vitro* method for determining the efficacy of a medical regimen in a patient already diagnosed with lung cancer comprising: a) measuring the levels of C4c fragment and prolactin, C4c fragment and CYFRA 21-1, C4c fragment and CRP, C4c fragment and prolactin and CYFRA 21-1, C4c fragment and prolactin and CRP, or C4c fragment and CYFRA 21-1 and CRP, or C4c fragment and CYFRA 21-1 and CRP and prolactin, in a sample obtained from the patient prior to the administration of the medical regimen; and b) measuring the levels of said biomarkers in a sample from the patient once started the administration of the medical regimen; and c) comparing the levels measured in steps (a) and (b), in such a way that if the biomarker levels measured in step (b) are lower than the levels measured in step (a), it is indicative that the medical regimen is effective in the treatment of lung cancer. In a preferred embodiment of the invention lung cancer is selected from the group consisting of non-small cell lung cancer and small-cell lung carcinoma. In a preferred embodiment of the invention the sample is selected from: blood, plasma, serum, bronchoalveolar lavage fluid, sputum, biopsy and surgical specimens. A plasma sample is particularly preferred.

The fourth embodiment of the present invention refers to an *in vitro* method for determining the efficacy of a medical regimen in a patient already diagnosed with lung cancer comprising: a) measuring the level of the C4c fragment in a sample from the patient once started the administration of the medical regimen; and b) comparing the C4c fragment level measured in step (a) with a reference control level of the C4c fragment or with the level of the C4c fragment in a sample from the same patient, under the same medical regimen, taken in a moment of time later than the sample taken in step (a), and c) wherein, if the C4c fragment level measured in step a) is not higher than the level measured in step (b), it is indicative that the medical regimen is effective in the treatment of lung cancer. In a preferred embodiment the present invention refers to an *in vitro* method for determining the efficacy of a medical regimen in a patient already diagnosed with lung cancer comprising: a) measuring the levels of C4c fragment and prolactin, C4c fragment and CYFRA 21-1, C4c fragment and CRP, C4c fragment and prolactin and CYFRA 21-1, C4c fragment and prolactin and CRP, or C4c fragment and CYFRA 21-1 and CRP, or C4c fragment and CYFRA 21-1 and CRP and prolactin, in a sample from the patient once started the administration of the medical regimen; and b) comparing the levels measured in step (a) with reference control levels of the biomarkers or with the levels of the C4c fragment in a sample from the same patient, under the same medical regimen, taken in a moment of time later than the sample taken in step (a), and c) wherein, if the biomarker levels measured in step a) are not higher than the level measured in step (b), it is indicative that the medical regimen is effective in the treatment of lung cancer. In a preferred embodiment of the invention lung cancer is selected from the group consisting of non-small cell lung cancer and small-cell lung carcinoma. In a preferred embodiment of the invention the sample is selected from: blood, plasma, serum, bronchoalveolar lavage fluid, sputum, biopsy and surgical specimens. A plasma sample is particularly preferred.

The fifth embodiment of the invention refers to the use of at least C4c fragment in the *in vitro* diagnosis of lung cancer or for the *in vitro* determination of whether an indeterminate pulmonary nodule, as identified for example by using an image technique (such as CT-scan), is or not malignant. In a preferred embodiment the present invention refers to the use of a combination of biomarkers comprising C4c fragment and prolactin, C4c fragment and CYFRA 21-1, C4c fragment and CRP, C4c fragment and prolactin and CYFRA 21-1, C4c fragment and prolactin and CRP, or C4c fragment and CYFRA 21-1 and CRP, or C4c fragment and CYFRA 21-1 and CRP and prolactin, in a method for the *in vitro* diagnosis of lung cancer or for the *in vitro* determination of whether an indeterminate pulmonary nodule, as identified by using an image techniques (such as CT-scan), is or not malignant. In a preferred embodiment of the invention lung cancer is selected from the group consisting of non-small cell lung cancer and small-cell lung carcinoma. In a preferred embodiment of the invention the sample is selected from: blood, plasma, serum, bronchoalveolar lavage fluid, sputum, biopsy and surgical specimens. A plasma sample is particularly preferred.

As explained above, it is important to note that image techniques (such as CT-scan) are commonly used today for diagnosing lung cancer and that said image techniques are associated with a high percentage of false positives, particularly in the case of indeterminate nodules detection. Unfortunately, current tools to discriminate benign from malignant nodules are suboptimal, and cancer research has not yet accomplished the goal of producing a marker that can be routinely used in the clinic. Consequently, a high percentage of patients are wrongly diagnosed as suffering from lung cancer and they are submitted to unnecessary invasive procedures which increase the cost of the Health Government System.

Consequently, a preferred embodiment of the present invention is directed to imaging techniques supplemented with the diagnostic/prognostic information obtained from the biomarkers as disclosed in embodiments one to five of the present invention. It is emphasized that this embodiment requires imaging a patient and the information obtained from said image technique being complemented with the information provided by the biomarkers as disclosed in embodiments one to five of the present invention, such combination of features amounts to significantly more than a natural phenomenon or abstract idea. Since measuring the level of said biomarkers comprising the C4c fragment offers greater sensitivity and specificity than commonly used image techniques, the percentage of false positives is reduced. Consequently the number of invasive techniques that are usually performed once a positive result is obtained from the image techniques is also reduced. Thus, the present invention offers significantly more as compared with the current standard for the diagnosis of lung cancer since the combination of the image technique with the level of said biomarkers comprising C4c improves the diagnosis of a patient at risk of having lung cancer who has been misdiagnosed with prior image techniques or of a patient known to have an indeterminate pulmonary nodule.

Consequently, the sixth embodiment of the invention is directed to an *in vitro* method for the diagnosis of lung cancer in a subject comprising: imaging the subject for lung cancer; and a) determining the level of at least the C4c fragment in a blood sample (plasma or serum) from the subject by contacting the test sample with a reagent that selectively binds the C4c fragment; and b) comparing the C4c fragment level determined in step a) with a reference control level of said C4c fragment, and c) wherein the imaging and the determination step (a) can be taken in any order and wherein if the C4c fragment level determined in step (a) is higher than the reference control level, it is indicative that the subject suffers from lung cancer and/or that the pulmonary nodules present in the subject are deemed to be malignant.

For the purpose of the present invention the following definitions are provided:
- The term "*comprising*" it is meant including, but not limited to, whatever follows the word "comprising". Thus, use of the term "comprising" indicates that the listed elements are required or mandatory, but that other elements are optional and may or may not be present.
- By "*consisting of"* is meant including, and limited to, whatever follows the phrase "consisting of". Thus, the phrase "consisting of" indicates that the listed elements are required or mandatory, and that no other elements may be present.
- The term "*reference*" or "*control level*", when referring to the level of the C4c fragment described in the present invention, refers to the level observed in patients not suffering from lung cancer. The patient is likely to suffer from lung cancer with a given sensitivity and specificity if the levels of C4c in the patient are above said "*reference*" or "*control level*"*.*

### Brief description of the figures

**Figure 1****.** Structure of the complement component C4 and its proteolytic fragments.
**Figure 2****.** Quantification of C4d (A) and C4c (B) in plasma samples from early stage lung cancer patients and control individuals.
**Figure 3****.** Correlation between C4d and C4c plasma levels in both control individuals and early stage lung cancer patients (A), only control individuals (B) or only lung cancer patients (C).
**Figure 4****.** ROC curves obtained from the quantification of C4d (A) and C4c (B) in plasma samples from patients with early lung cancer and control individuals.
**Figure 5****.** Quantification of 24 cancer-related biomarkers in plasma samples from early stage lung cancer patients and control individuals measured by Luminex technology.
**Figure 6****.** Quantification of IL6 (A), prolactin (B), CYFRA 21-1 (C) and CRP in plasma samples from early stage lung cancer patients and control individuals using Cobas technology, and correlation of the levels of IL6 (A), prolactin (B) and CYFRA 21-1 (C) as measured using Luminex and Cobas technologies. Of note, no correlation is shown for CRP because this marker was not determined by Luminex technology.
**Figure 7****.** ROC curve obtained from the diagnostic model based on the quantification of C4c, prolactin, CYFRA 21-1 and CRP in plasma samples from patients with early lung cancer and control individuals.
**Figure 8****.** ROC curves from the quantification of C4c (A) and C4c/CYFRA 21-1/CRP (B) in plasma samples from patients with malignant and non-malignant pulmonary nodules.
**Figure 9****.** ROC curves for the discrimination between benign and malignant lung nodules obtained using a diagnostic model based on C4c quantification in plasma samples and the clinical features age, smoking status, smoking history and nodule size (A), or based on the quantification of C4c, CYFRA 21-1 and CRP in plasma samples and the same clinical features (B).
**Figure 10****.** ROC curves for the discrimination between benign and malignant lung nodules obtained using the Gould's diagnostic model (A), a model based on the Gould's model and C4c quantification in plasma (B), or a model based on the Gould's model and the quantification of C4c, CYFRA21-1 and CRP in plasma (C).

### Detailed description of the invention

**Example 1.** C4c has a better performance than C4d in the diagnosis of lung cancer.

### Description of the experiment

C4c and C4d-containing fragments (from now on referred as C4d) were determined in plasma samples from early stage lung cancer patients and control individuals.

### Material and methods

Plasma samples from 39 patients with surgically resectable lung cancer at early stages (I and II) and from 39 healthy people (matched by sex, age and smoking history) were obtained at the Clinica Universidad de Navarra. A summary of the characteristics of these patients and controls is shown in **Table 1.** Lung tumors were classified according to the World Health Organization 2004 classification.

**Table 1. Epidemiological and clinical characteristics of cases and controls.**

| **Characteristics** | **Control individuals** | **Lung cancer patients** |
|---|---|---|
| **Sex** | | |
| Male | 34 | 34 |
| Female | 5 | 5 |
| **Age** | | |
| ≤70 years | 30 | 31 |
| >70 years | 9 | 8 |
| **Smoking status** | | |
| Ex-smoker* | 27 | 26 |
| Current smoker | 12 | 13 |
| **Histology** | | |
| Adenocarcinoma | | 19 |
| Squamous cell carcinoma | | 20 |
| **Nodule size** | | |
| ≤3 cm | | 22 |
| >3 cm | | 17 |
| **Stage** | | |
| I | | 31 |
| II | | 8 |

| | | |
|---|---|---|
| *Also including one never smoker | | |

Plasma samples were spun down at 300 g for 10 min, and the supernatants were collected. Samples were stored at -80°C until analysis. C4d was determined using a commercially-available enzyme-linked immunosorbent assay (A008, Quidel). The assay recognizes all C4d-containing fragments of activated C4 (including C4b, iC4b and/or C4d). C4d-containing fragments of activated C4 are referred in this document as C4d. C4c levels were determined as previously described and expressed as arbitrary units [Pilely K, Skjoedt MO, Nielsen C, Andersen TE, Louise Aabom A, Vitved L, Koch C, Skjødt K, Palarasah Y. A specific assay for quantification of human C4c by use of an anti-C4c monoclonal antibody. J Immunol Methods 2014; 405: 87-96*].*

Receiver operating characteristic (ROC) curves were generated in order to evaluate the diagnostic performance of the biomarkers. Other parameters such as sensitivity, specificity, positive predictive value, negative predictive value, likelihood positive ratio and likelihood negative ratio were also assessed. Statistical analyses were carried out with STATA/IC 12.1.

### Results

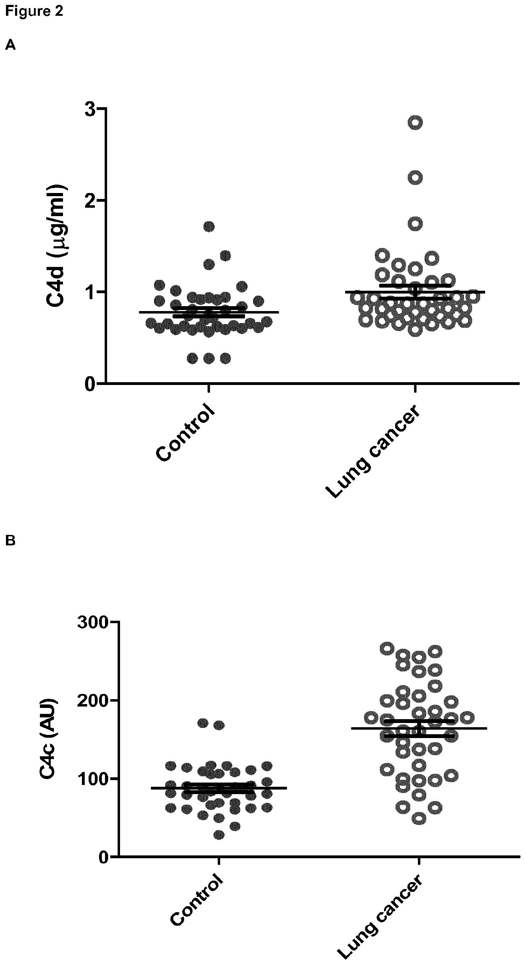

Levels of C4d in plasma samples from control individuals and lung cancer patients were 0.79±0.28 µg/ml vs. 1.00±0.46 µg/ml, respectively (**Figure 2A**). In the case of C4c, plasma levels were 88±30 AU in control individuals, and 164±60 AU in lung cancer patients (**Figure 2B**). Associations between the levels of these two molecular markers and sex, age, smoking status, histology, nodule size and stage are shown in **Table 2** and **Table 3.**

**Table 2. Association between C4d plasma levels and clinicopathological features of lung cancer cases and controls.**

| | **Controls** | **Cases** | | |
|---|---|---|---|---|
| **Characteristics** | **C4d (µg/ml)** | **p value¹** | **C4d (µg/ml)** | **p value¹** |
| **Sex** | | | | |
| Male | 0.78±0.30 | 0.737 | 1.03±0.47 | 0.172 |
| Female | 0.77±0.09 | | 0.79±0.11 | |
| **Age** (years) | | | | |
| ≤70 | 0.75±0.28 | 0.257 | 1.02±0.48 | 0.767 |
| >70 | 0.89±0.30 | | 0.91±0.24 | |
| **Smoking status** | | | | |
| Ex-smoker | 0.73±0.24 | 0.361 | 1.04±0.50 | 0.290 |
| Current smoker | 0.88±0.35 | | 0.91±0.32 | |
| **Histology** | | | | |
| Adenocarcinoma | | | 0.88±0.20 | 0.221 |
| Squamous cell carcinoma | | | 1.11±0.57 | |
| **Nodule size** | | | | |
| ≤3 cm | | | 0.90±0.29 | 0.130 |
| >3 cm | | | 1.13±0.58 | |
| **Stage** | | | | |
| I | | | 0.93±0.34 | 0.044 |
| II | | | 1.28±0.69 | |

| | | | | |
|---|---|---|---|---|
| ¹Mann-Whitney U test | | | | |

**Table 3. Association between C4c plasma levels and clinicopathological features of lung cancer cases and controls.**

| | **Controls** | | **Cases** | |
|---|---|---|---|---|
| **Characteristics** | **C4c (AU)¹** | **p value2** | **C4c (AU)¹** | **p value²** |
| **Sex** | | | | |
| Male | 89±31 | 0.801 | 166±63 | 0.614 |
| Female | 83±21 | | 154±34 | |
| **Age** (years) | | | | |
| ≤70 | 84±28 | 0.243 | 162±61 | 0.835 |
| >70 | 100±32 | | 170±58 | |
| **Smoking status** | | | | |
| Ex-smoker | 87±30 | 0.831 | 162±65 | 0.882 |
| Current smoker | 91±30 | | 167±52 | |
| **Histology** | | | | |
| Adenocarcinoma | | | 157±54 | 0.431 |
| Squamous cell carcinoma | | | 171±66 | |
| **Nodule size** | | | | |
| ≤3 cm | | | 155±65 | 0.223 |
| >3 cm | | | 176±53 | |
| **Stage** | | | | |
| I | | | 159±58 | 0.251 |
| II | | | 184±66 | |

| | | | | |
|---|---|---|---|---|
| ¹AU: Arbitrary units. ²Mann-Whitney U test | | | | |

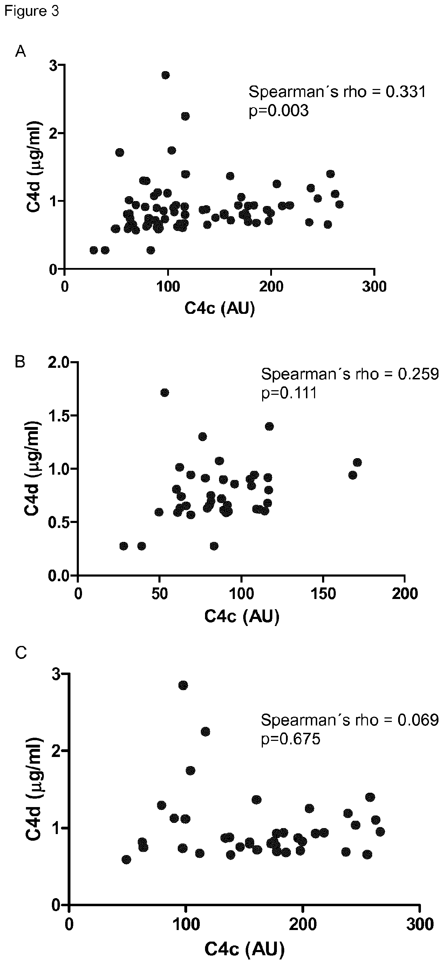

Correlation studies showed that the quantification of C4d and the quantification of C4c were not equivalent. Thus, only a weak association was observed between both markers when all samples were analyzed together (**Figure 3A**). Moreover, this association disappeared when controls and cases were analyzed separately (**Figure 3B-C**).

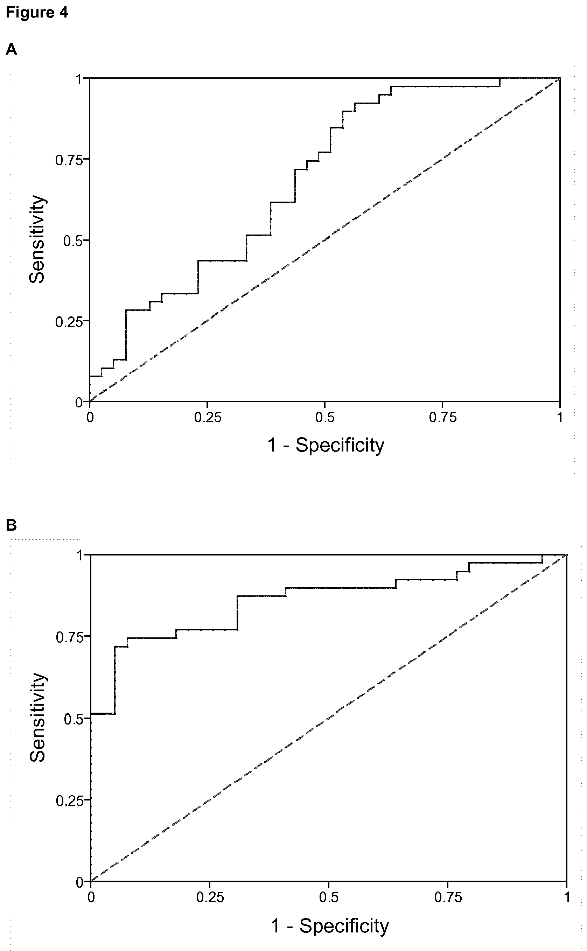

To evaluate the capacity of the markers to discriminate between cases and controls, ROC curves were generated (**Figure 4**). Areas under the ROC curve were 0.69 (95% CI=0.57-0.81) for C4d and 0.86 (95% CI=0.77-0.95) for C4c. The area under the curve for C4c was significantly better than that for C4d (p=0.001). Likewise, the performance of C4c was superior to that of C4d in all other diagnostic characteristics analyzed (**Table 4**).

**Table 4. Diagnostic performance of the determination of C4d and C4c levels in plasma samples from early stage lung cancer patients.**

| | C4d | C4c |
|---|---|---|
| Sensitivity | 51% | 78% |
| Specificity | 67% | 95% |
| Positive predictive value | 61% | 93% |
| Negative predictive value | 58% | 77% |
| Likelihood positive ratio | 1.54 | 14.00 |
| Likelihood negative ratio | 0.73 | 0.30 |
| Correctly classified | 59% | 83% |

### Conclusion

This analysis evidences that plasma samples taken from patients with lung cancer at early stages (I and II) contain higher levels of C4c than plasma samples taken from control individuals. Moreover, the determination of this biomarker has a significantly superior diagnostic performance than the determination of C4d, another proteolytic fragment derived from complement activation previously proposed as a diagnostic marker for lung cancer.

**Example 2.** Combination of C4c with other cancer markers increases its diagnostic potential.

### Description of the experiment

The diagnostic performance of several cancer markers was evaluated in the set of patients described in Example 1. The diagnostic information provided by the quantification of some of these markers was used to improve the diagnostic accuracy of C4c.

### Material and methods

Epidemiological and clinical characteristics of the early stage lung cancer patients are described in Example 1. The analytical evaluation of the cancer markers was performed in plasma samples using the Human Circulating Cancer Biomarkers Magnetic Bead Panel 1 (HCCBP1 MAG-58K, Millipore) with Luminex technology. The markers analyzed were: AFP, total PSA, CA15-3, CA19-9, MIF, TRAIL, leptin, IL-6, sFasL, CEA, CA125, IL-8, HGF, sFas, TNFα, prolactin, SCF, CYFRA 21-1, OPN, FGF2, β-HCG, HE4, TGFα, VEGF. A Cobas analyzer (Roche) was also used for the determination of IL6, prolactin, CYFRA 21-1 and CRP. Logistic regression was used to generate the diagnostic models.

### Results

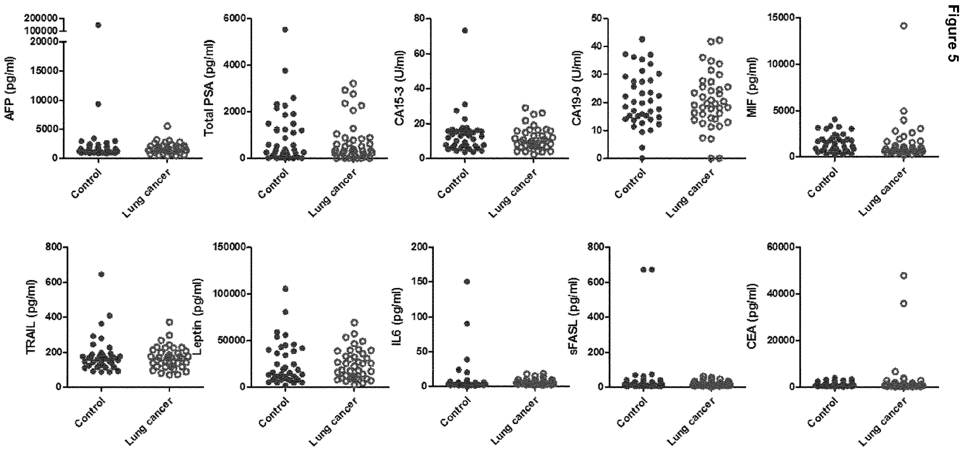

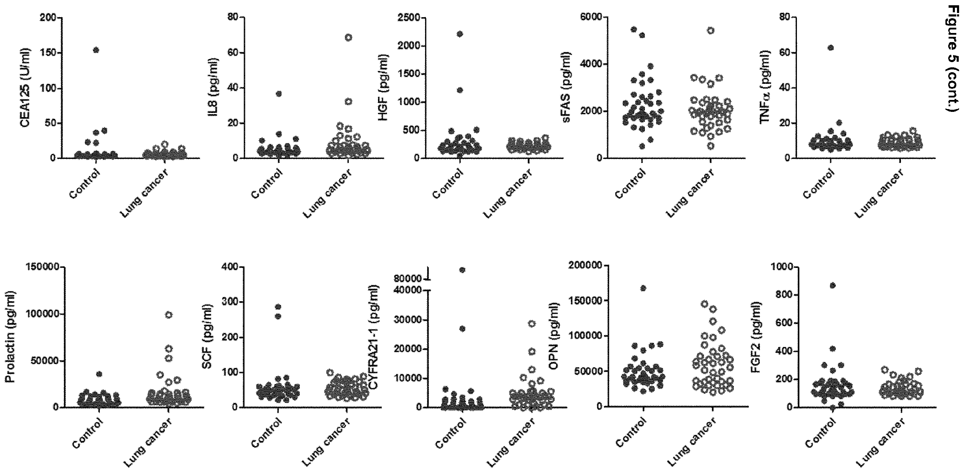

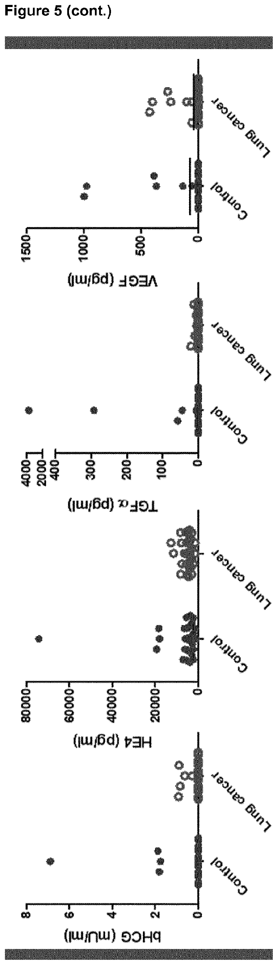

Results from the evaluation of the 24 potential diagnostic markers with Luminex technology are shown in **Table 5** and **Figure 5****.** Significant differences between controls and cases were found in the plasma levels of IL6, prolactin and CYFRA 21-1.

**Table 5. Marker levels (mean±SD) in plasma samples from control individuals and lung cancer patients at early stages, as determined by Luminex technology.**

| **Marker** | **Controls** | **Cases** | **p value¹** |
|---|---|---|---|
| AFP (pg/ml) | 5509±23409 | 1696±951 | 0.893 |
| Total PSA (pg/ml) | 956±1174 | 714±888 | 0.475 |
| CA15-3 (U/ml) | 13.3±11.7 | 11.2±6.5 | 0.549 |
| CA19-9 (U/ml) | 21.0±9.8 | 20.5±9.8 | 0.877 |
| MIF (pg/ml) | 1526±1002 | 1550±2314 | 0.124 |
| TRAIL (pg/ml) | 183±102 | 168±63 | 0.916 |
| Leptin (pg/ml) | 25304±22950 | 24553±16283 | 0.586 |
| IL6 (pg/ml) | 11.8±27.4 | 6.9±4.1 | 0.022 |
| sFASL (pg/ml) | 56.3±146 | 21.3±14.5 | 0.455 |
| CEA (pg/ml) | 1326±986 | 3382±9251 | 0.738 |
| CA125 (U/ml) | 11.1±25.0 | 6.2±3.5 | 0.319 |
| IL8 (pg/ml) | 5.8±5.6 | 8.4±11.3 | 0.254 |
| HGF (pg/ml) | 300±365 | 228±53 | 0.340 |
| sFAS (pg/ml) | 2273±1018 | 2054±855 | 0.463 |
| TNFα (pg/ml) | 10.4±9.1 | 9.1±2.4 | 0.996 |
| Prolactin (pg/ml) | 8759±5885 | 16442±18237 | 0.032 |
| SCF (pg/ml) | 59.8±52.4 | 54.0±19.1 | 0.569 |
| CYFRA 21-1 (pg/ml) | 1294±537 | 3797±5529 | <0.001 |
| OPN (pg/ml) | 50181±25313 | 59616±31881 | 0.310 |
| FGF2 (pg/ml) | 162±140 | 138±48.9 | 0.798 |
| bHCG (mU/ml) | 0.32±1.2 | 0.09±0.26 | 0.864 |
| HE4 (pg/ml) | 7148±11785 | 5036±2303 | 0.860 |
| TGFα (pg/ml) | 106±597 | 1.5±4.2 | 0.890 |
| VEGF (pg/ml) | 74.9±231 | 40±106 | 0.847 |

| | | | |
|---|---|---|---|
| ¹Mann-Whitney U test | | | |

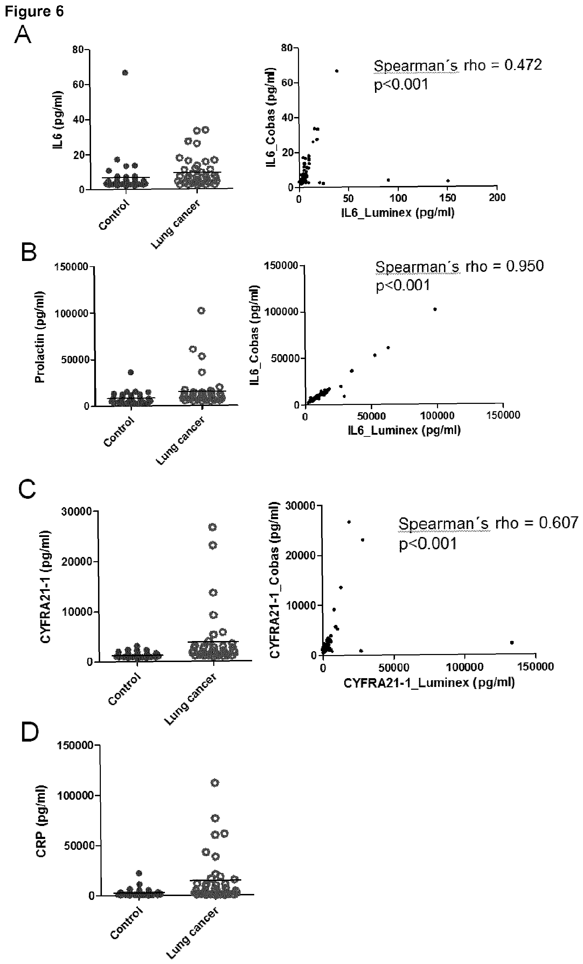

We next validated the results obtained for IL6, prolactin and CYFRA 21-1 using Cobas technology. Of note, CYFRA 21-1 could not be determined in one control sample. Correlations between Luminex and Cobas technologies were also evaluated for each of these markers. Additionally, we analyzed the levels of CRP, a biomarker that was not present in the Millipore panel. Results of these analyses are showed in **Table 6** and **Figure 6****.** All markers showed significant differences between cases and controls.

**Table 6. Marker levels (mean±SD) in plasma samples from control individuals and lung cancer patients at early stages, as determined by Cobas technology.**

| **Marker** | **Controls** | **Cases** | **p value¹** |
|---|---|---|---|
| IL6 (pg/ml) | 6.7±10.4 | 9.5±8.3 | 0.015 |
| Prolactin (pg/ml) | 8330±5970 | 15389±18385 | 0.003 |
| CYFRA 21-1 (pg/ml) | 1294±537 | 3797±5529 | <0.001 |
| CRP (pg/ml) | 2803±3844 | 14692±24412 | 0.004 |

| | | | |
|---|---|---|---|
| ¹Mann-Whitney U test | | | |

The next objective was to develop diagnostic models using combinations of the levels of C4c and these four markers (IL6, prolactin, CYFRA 21-1 and CRP). For that, in first place, a univariate analysis was performed for each of the markers to determine their individual predictive capacity. As shown in **Table 7,** IL6 did not predicted malignancy and, therefore, was not included in the multivariate analysis.

**Table 7. Simple logistic regression for the evaluation of C4c, prolactin, CYFRA 21-1 and CRP as potential diagnostic markers in lung cancer.**

| **Marker** | **LR chi²** | **p value** |
|---|---|---|
| C4c | 39.18 | <0.001 |
| IL6 | 1.80 | 0.180 |
| Prolactin | 7.40 | 0.006 |
| CYFRA 21-1 | 24.38 | <0.001 |
| CRP | 14.52 | <0.001 |

| | | |
|---|---|---|
| LR chi²: likelihood ratio chi-square test | | |

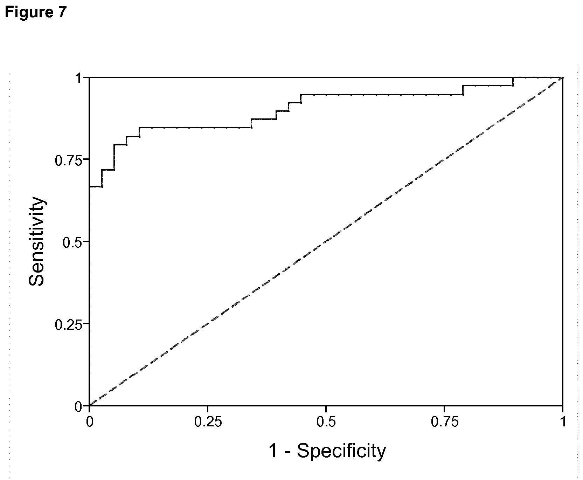

A mulitivariate model was developed using the plasma levels of C4c, prolactin, CYFRA 21-1 and CRP. Of note, the individual in whom CYFRA 21-1 could not be determined was removed from the multivariate analysis. Multivariate logistic regression analysis generated a model with a value of 52.09 for the likelihood ratio chi-square test (p<0.001). The predicted probabilities of the model were compared with the final diagnoses, and a ROC curve was constructed. The area under the ROC curve was 0.91 (95% CI=0.83-0.98) (**Figure 7**). Other diagnostic characteristics of the model are shown in **Table 7.** This multivariate diagnostic model including the four markers was able to correctly classify a higher percentage of subjects than C4c alone (87% vs 83%).

**Table 7. Performance of the lung cancer diagnostic model based on the plasma levels of C4c, prolactin, CYFRA 21-1 and CRP.**

| | C4c/Prolactin/CYFRA 21-1/CRP |
|---|---|
| Sensitivity | 82% |
| Specificity | 92% |
| Positive predictive value | 91% |
| Negative predictive value | 83% |
| Likelihood positive ratio | 10.39 |
| Likelihood negative ratio | 0.19 |
| Correctly classified | 87% |

Associations between the predictive probabilities of malignancy derived from the model and clinicopathological characteristics of the patients and controls are shown in **Table 8.**

**Table 8. Association between the probabilities obtained from the multivariate model, based on C4c/Prolactin/CYFRA 21-1/CRP plasma levels, and the characteristics of lung cancer patients and controls.**

| | **Controls** | | **Cases** | |
|---|---|---|---|---|
| **Characteristics** | **Score** | **p value¹** | **Score** | **p value¹** |
| **Sex** | | | | |
| Male | 0.23±0.19 | 0.915 | 0.78±0.32 | 0.166 |
| Female | 0.22±0.16 | | 0.76±0.17 | |
| **Age** (years) | | | | |
| ≤65 | 0.18±0.19 | 0.015 | 0.74±0.32 | 0.237 |
| >65 | 0.28±0.17 | | 0.82±0.28 | |
| **Smoking status** | | | | |
| Ex-smoker | 0.23±0.19 | 0.730 | 0.79±0.30 | 0.766 |
| Current smoker | 0.22±0.19 | | 0.77±0.31 | |
| **Histology** | | | | |
| Adenocarcinoma | | | 0.69±0.31 | 0.012 |
| Squamous cell carcinoma | | | 0.86±0.26 | |
| **Nodule size** | | | | |
| ≤3 cm | | | 0.70±0.33 | 0.019 |
| >3 cm | | | 0.89±0.22 | |
| **Stage** | | | | |
| I | | | 0.76±0.31 | 0.186 |
| II | | | 0.86±0.25 | |

| | | | | |
|---|---|---|---|---|
| ¹Mann-Whitney U test | | | | |

Multivariate models based on C4c and different combinations of the other three markers were also generated. **Table 9** shows areas under the ROC curves and percentages of correctly classified events from these combinations. In most cases, the combined models improved the diagnostic characteristics of C4c alone.

**Table 9. C4c-based diagnostic models generated by logistic regression using different combinations of C4c and prolactin, CYFRA 21-1 and/or CRP.**

| **Markers in the model** | **Area under the curve** | **Correctly classified events** |
|---|---|---|
| C4c | 0.87 (95% CI=0.77-0.95) | 83% |
| C4c/Prolactin | 0.87 (95% CI=0.79-0.95) | 82% |
| C4c/ CYFRA 21-1 | 0.91 (95% CI=0.84-0.98) | 87% |
| C4c/CRP | 0.87 (95% CI=0.78-0.95) | 85% |
| C4c/Prolactin/CYFRA 21-1 | 0.90 (95% CI=0.83-0.98) | 87% |
| C4c/Prolactin/CRP | 0.87 (95% CI=0.79-0.95) | 85% |
| C4c/CYFRA 21-1/CRP | 0.91 (95% CI=0.83-0.98) | 87% |

### Conclusion

These analyses evidence the capacity to diagnose lung cancer of models based on the combination of plasma levels of C4c with prolactin and/or CYFRA 21-1 and/or CRP.

**Example 3.** Determination of C4c in combination with other protein markers can be used to discriminate benign from malignant indeterminate pulmonary nodules.

### Description of the experiment

The capacity of the C4c-based models to discriminate between patients with and without lung cancer was evaluated in a set of plasma samples from patients presenting benign or malignant lung nodules discovered by chest CT.

### Material and methods

A set of plasma samples from Vanderbilt University Medical Center was used in the study. This cohort included plasma samples from 138 patients presenting indeterminate lung nodules discovered by chest CT. Lung nodules were defined as rounded opacities completely surrounded by lung parenchyma. Seventy six indeterminate lung nodules were diagnosed as lung cancers by pathological examination, whereas the remaining 62 were diagnosed as non-malignant. Clinical features of malignant and non-malignant nodules are shown in **Table 10.** Diagnosis of non-malignant nodules included lung lesions such as chronic obstructive pulmonary disease, emphysema, inflammatory disease, granulomatous lesions, and hamartomas.

**Table 10. Clinical and epidemiological features in the set of patients with indeterminate pulmonary nodules.**

| **Characteristics** | **Non-malignant lung nodules** | **Malignant-lung nodules** |
|---|---|---|
| **Sex** | | |
| Male | 36 | 50 |
| Female | 26 | 26 |
| **Age** | | |
| ≤65 | 46 | 33 |
| >65 | 16 | 43 |
| **Smoking status** | | |
| Never | 16 | 2 |
| Former | 24 | 42 |
| Current | 22 | 32 |
| **Pack-years** | | |
| ≤50 | 45 | 35 |
| >50 | 17 | 41 |
| **Nodule size** | | |
| ≤3 cm | 46 | 30 |
| >3 cm | 12 | 46 |
| Not available | 4 | |
| **FEV1% predicted** | | |
| ≤80 | 29 | 50 |
| >80 | 17 | 12 |
| Not available | 16 | |
| **Histology¹** | | |
| ADC | | 26 |
| SCC | | 16 |
| LCC | | 6 |
| SCLC | | 15 |
| NSCLC NOS | | 13 |
| **Stage** | | |
| I-II | | 17 |
| III-IV | | 39 |
| Not available | | 20 |
| **Status** | | |
| Alive | | 23 |
| Death | | 53 |

| | | |
|---|---|---|
| ¹ADC: Adenocarcinoma; SCC: Squamous cell carcinoma; LCC: Large cell carcinoma; SCLC: Small cell carcinoma; NSCLC NOS: Non-small cell lung cancer not otherwise specified. | | |

Prolactin, CYFRA 21-1 and CRP plasma levels were analyzed using Cobas technology (Roche). C4d was evaluated as indicated in Example 1. C4c was evaluated using an enzyme-linked immunosorbent assay. Briefly, 96 well plates were coated with 200 ng of capture antibody (antiC4c antibody; ref. CAM072-18, BioPorto Diagnostics). Plates were washed with wash buffer (PBS, 0.05% Tween-20, pH=7.4), and blocked with the same buffer. Samples were diluted 1:8 in assay buffer (wash buffer containing 20 mM EDTA). A plasma sample from a healthy individual, diluted 1:2, 1:4, 1:8 and 1:16, was used as a reference for quantification. After 1 hour at room temperature, plates were washed and a detection antibody was added (1:50,000; biotinylated antiC4b antibody; ref. HYB162-02B, BioPorto Diagnostics). Plates were washed and developed with streptavidin (1:200) and Substrate Reagent Pack (ref. DY999; R&D Systems). Results were calculated as relative values to those found in the reference sample and expressed as arbitrary units (AU). Logistic regression was used to generate the diagnostic models.

### Results

Plasma C4c levels were significantly higher in lung cancer patients than in their non-lung cancer counterparts (9.89±3.95 vs 7.17±5.16 AU; p=0.002). The area under the ROC curve was 0.66 (95% CI=0.56 to 0.75) (**Figure 8A**). In the case of C4d, there were no statistical differences in the plasma levels of this marker between individuals with benign and malignant nodules (4.60±2.06 µg/ml vs. 5.09±2.10 µg/ml; p=0.212), suggesting that the determination of C4d-containing fragments of activated C4 is ineffective in this clinical context. The diagnostic performance of C4c is summarized in **Table 11.**

**Table 11. Performance of the determination of C4c levels in plasma samples as a potential diagnostic marker for the discrimination between benign and malignant pulmonary nodules.**

| | C4c |
|---|---|
| Sensitivity | 88% |
| Specificity | 44% |
| Positive predictive value | 6% |
| Negative predictive value | 98.9% |
| Likelihood positive ratio | 1.56 |
| Likelihood negative ratio | 0.27 |
| Correctly classified | 68% |

| | |
|---|---|
| ¹Positive and negative predictive values were calculated with an estimated prevalence of malignant nodules in this clinical setting of 4%. | |

Associations between C4c levels and epidemiological and clinical characteristics of patients are shown in **Table 12.**

**Table 12. Association between C4c plasma levels and clinical features in the set of patients with indeterminate pulmonary nodules.**

| | **Benign** | | **Malignant** | |
|---|---|---|---|---|
| **Characteristics** | **C4c (AU)¹** | **p value²** | **C4c (AU)¹** | **p value²** |
| **Sex** | | | | |
| Male | 6.72±4.12 | 0.663 | 9.50±3.17 | 0.206 |
| Female | 7.78±6.36 | | 10.68±5.11 | |
| **Age** (years) | | | | |
| ≤65 | 8.11±5.46 | 0.398 | 10.70±4.79 | 0.753 |
| >65 | 6.76±4.02 | | 9.45±3.37 | |
| **Smoking status** | | | | |
| Never smoker | 9.13±6.80 | 0.212 | 11.57±1.04 | 0.364 |
| Former/current smoker | 6.48±4.34 | | 9.85±3.99 | |
| **Pack-years** | | | | |
| ≤50 | 7.08±5.59 | 0.795 | 9.38±3.40 | 0.823 |
| >50 | 7.39±3.64 | | 10.34±4.36 | |
| **Nodule size** | | | | |
| ≤3 cm | 7.76±5.21 | 0.219 | 9.71±3.37 | 0.807 |
| >3 cm | 6.02±4.93 | | 10.01±4.32 | |
| **FEV1% predicted** | | | | |
| ≤80 | 5.96±4.13 | 0.700 | 10.08±4.28 | 0.430 |
| >80 | 6.44±7.29 | | 10.43±3.96 | |
| **Histology³** | | | | |
| ADC | | | 11.55±4.25 | <0.001 |
| SCC | | | 7.51±2.87 | |
| LCC | | | 12.52±4.74 | |
| SCLC | | | 8.04±2.70 | |
| NSCLC NOS | | | 10.45±3.18 | |
| **Stage** | | | | |
| I-II | | | 11.32±4.88 | 0.964 |
| III-IV | | | 10.25±3.84 | |
| **Status** | | | | |
| Alive | | | 10.96±4.19 | 0.442 |
| Death | | | 9.43±3.79 | |

| | | | | |
|---|---|---|---|---|
| ¹Arbitrary units. ¹Mann-Whitney U test or Kruskal Wallis test.³ADC: Adenocarcinoma; SCC: Squamous cell carcinoma; LCC: Large cell carcinoma; SCLC: Small cell carcinoma; NSCLC NOS: Non-small cell lung cancer not otherwise specified. | | | | |

The next objective was to develop diagnostic models using the combined information provided by C4c and the three protein markers found differentially expressed between cases and controls in Example 2 (prolactin, CYFRA 21-1 and CRP). A logistic regression analysis performed for each of the markers individually is shown in **Table 13.**

**Table 13. Univariate logistic regression for the evaluation of C4c, prolactin, CYFRA 21-1 and CRP as potential diagnostic markers for the discrimination between benign and malignant pulmonary nodules.**

| **Marker** | **LR chi²** | **p value** |
|---|---|---|
| C4c | 12.11 | <0.001 |
| Prolactin | 2.54 | 0.111 |
| CYFRA 21-1 | 45.12 | <0.001 |
| CRP | 19.87 | <0.001 |

Based on the univariate analyses, C4c, CYFRA 21-1 and CRP were predictors of malignancy and were included in the multivariate logistic regression analysis. This study generated a model with a value of 64.04 for the likelihood ratio chi-square test (p<0.001). The predicted probabilities of the model were compared with the final diagnoses, and a ROC curve was constructed. The area under the curve was 0.86 (95% CI=0.80-0.92) (**Figure 8B**). Other diagnostic characteristics of the model are shown in **Table 14.**

**Table 14. Performance of the diagnostic model for the discrimination between benign and malignant pulmonary nodules based on plasma levels of C4c, CYFRA 21-1 and CRP.**

| | C4c/ CYFRA 21-1/CRP |
|---|---|
| Sensitivity | 75% |
| Specificity | 85% |
| Positive predictive value¹ | 18% |
| Negative predictive value¹ | 98.8% |
| Likelihood positive ratio | 5.17 |
| Likelihood negative ratio | 0.29 |
| Correctly classified | 80% |

| | |
|---|---|
| ¹Positive and negative predictive values were calculated with an estimated prevalence of malignant nodules in this clinical setting of 4%. | |

Associations between the predictive capacity of the model and characteristics of the patients are shown in **Table 15.** Interestingly, the predictive probabilities of malignancy were significantly associated with nodule size and vital status in the malignant group.

**Table 15. Association between the scores obtained from the multivariate model, based on C4c/CYFRA 21-1/CRP plasma levels, and the characteristics of patients with malignant lung nodules and controls with benign nodules.**

| | **Benign** | | **Malignant** | |
|---|---|---|---|---|
| **Characteristics** | **Diagnostic score (AU)¹** | **p value2** | **Diagnostic score (AU)¹** | **p value²** |
| **Sex** | | | | |
| Male | 0.33±0.19 | 0.887 | 0.74±0.27 | 0.466 |
| Female | 0.34±0.21 | | 0.70±0.28 | |
| **Age** (years) | | | | |
| ≤65 | 0.33±0.20 | 0.987 | 0.74±0.28 | 0.509 |
| >65 | 0.34±0.21 | | 0.71±0.26 | |
| **Smoking status** | | | | |
| Never smoker | 0.40±0.21 | 0.111 | 0.65±0.04 | 0.436 |
| Former/current smoker | 0.31±0.19 | | 0.73±0.27 | |
| **Pack-years** | | | | |
| ≤50 | 0.33±0.19 | 0.994 | 0.70±0.25 | 0.265 |
| >50 | 0.34±0.22 | | 0.75±0.28 | |
| **FEV1% predicted** | 0.32±0.18 | 0.309 | 0.72±0.28 | 0.178 |
| ≤80 | 0.27±0.20 | | 0.64±0.20 | |
| >80 | | | | |
| **Nodule size** | | | | |
| ≤3 cm | 0.34±0.20 | 0.863 | 0.58±0.29 | <0.001 |
| > 3 cm | 0.32±0.17 | | 0.83±0.21 | |
| **Histology** | | | | |
| ADC | | | 0.72±0.25 | 0.706 |
| SCC | | | 0.75±0.31 | |
| LCC | | | 0.66±0.32 | |
| SCLC | | | 0.65±0.30 | |
| NSCLC NOS | | | 0.85±0.15 | |
| **Stage** | | | | |
| I-II | | | 0.68±0.31 | 0.258 |
| III-IV | | | 0.77±0.23 | |
| **Status** | | | | |
| Alive | | | 0.70±0.26 | 0.005 |
| Death | | | 0.84±0.22 | |

| | | | | |
|---|---|---|---|---|
| ¹Arbitrary units. ²Mann-Whitney U test or Kruskal Wallis test. | | | | |

The next aim was to generate diagnostic models based on both molecular markers and clinical features. First, univariate logistic regression analyses were performed for these last features (**Table 16**).

**Table 16. Univariate logistic regression for the evaluation of clinical variables as markers for discrimination of pulmonary nodules.**

| **Marker** | **LR chi²** | **p value** |
|---|---|---|
| Sex | 0.87 | 0.352 |
| Age | 14.98 | <0.001 |
| Smoking status | 17.57 | <0.001 |
| Pack-years | 11.12 | <0.001 |
| FEV1% Predicted | 2.42 | 0.120 |
| Nodule size | 27.18 | <0.001 |

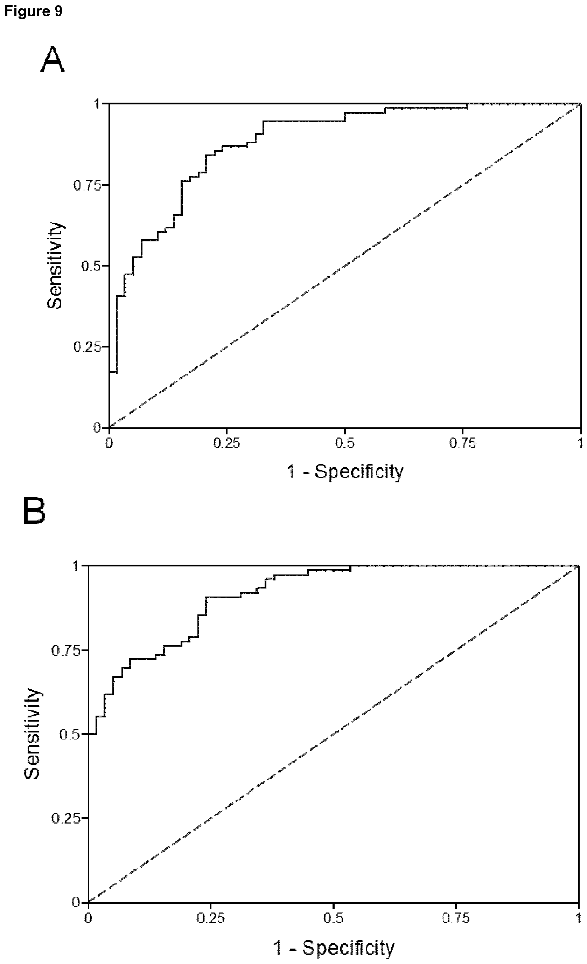

Based on the results obtained from these univariate analyses, clinical variables selected to be included in the model were age, smoking status, pack-years and nodule size. Two combined models were generated, one including the clinical variables and C4c, and another including the clinical variables and the three molecular markers. In the first case (C4c combined with clinical variables), the LR chi2 of the model was 69.81 (p<0.001). The area under the ROC curve was 0.88 (95% CI=0.83-0.94) (**Figure 9A**). The LR chi2 of the model based on the three molecular markers and the clinical variables was 86.82 (p<0.001). The area under the ROC curve was 0.92 (95% CI=0.87-0.96) (**Figure 9B**). Other diagnostic characteristics of the two models are shown in **Table 17.**

**Table 17. Performance of lung cancer diagnostic models based on the plasma levels of molecular markers (C4c alone, or together with CYFRA 21-1 and CRP) and clinical variables (age, smoking status, pack-years and nodule size).**

| | Clinical variables + C4c | Clinical variables + C4c/CYFRA 21-1/CRP |
|---|---|---|
| Sensitivity | 87% | 91% |
| Specificity | 76% | 76% |
| Positive predictive value¹ | 13% | 14% |
| Negative predictive value¹ | 99.3% | 99.5% |
| Likelihood positive ratio | 3.60 | 3.76 |
| Likelihood negative ratio | 0.17 | 0.12 |
| Correctly classified | 82% | 84% |

| | | |
|---|---|---|
| ¹Positive and negative predictive values were calculated with an estimated prevalence of malignant nodules in this clinical setting of 4%. | | |

Noninvasive diagnostic models for lung cancer based on clinical and image characteristics have been described. Gould et al. developed and validated a clinical model to discriminate lung cancer from benign lung nodules using age, smoking history, nodule diameter and time since quitting smoking. This model has been previously used to evaluate the diagnostic value added by molecular signatures to clinical and chest CT data for the noninvasive diagnosis of patients presenting indeterminate pulmonary nodules. Similarly, we evaluated the capacity of C4c, alone or in combination with CYFRA 21-1 and CRP, to complement this validated clinical model for the detection of lung cancer in patients with indeterminate pulmonary nodules.

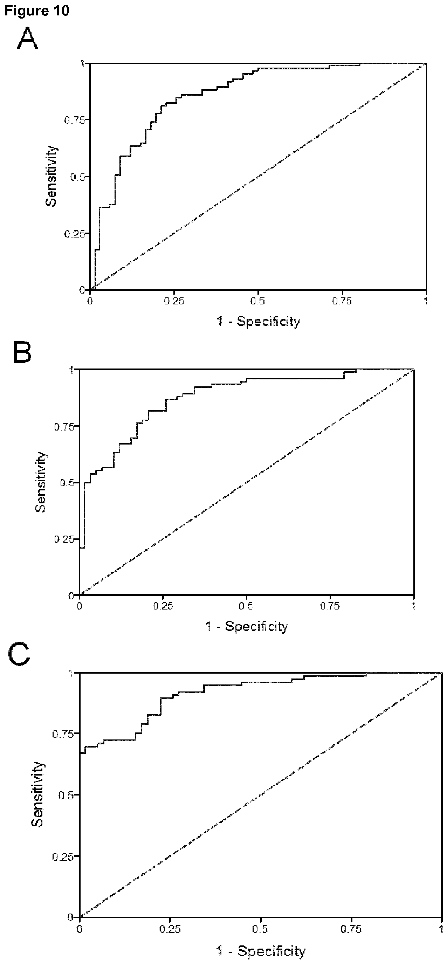

We first assessed the diagnostic performance of the Gould's clinical model in our set of patients. Due to the limited clinical information available from some patients, the model could only be applied to 134 patients. A logistic regression analysis yielded an LR chi2 of 58.19 (p<0.001). The area under the ROC curve was 0.86 (95% CI=0.80-0.92) (**Figure 10A**). Other diagnostic characteristics of the model are shown in **Table 18.**

**Table 18. Performance of the Gould's model based on the clinical variables age, smoking history, nodule diameter and time since quitting smoking.**

| | Gould's model |
|---|---|
| Sensitivity | 86% |
| Specificity | 73% |
| Positive predictive value¹ | 12% |
| Negative predictive value¹ | 99.2% |
| Likelihood positive ratio | 3.15 |
| Likelihood negative ratio | 0.20 |
| Correctly classified | 80% |

| | |
|---|---|
| ¹Positive and negative predictive values were calculated with an estimated prevalence of malignant nodules in this clinical setting of 4%. | |

The accuracy of the clinical model increased when C4c or C4c/CYFRA 21-1/CRP was added to the model. In the first case (Gould's model and C4c), the LR chi2 was 63.96 (p<0.001). The area under the ROC curve was 0.87 (95% CI=0.81-0.93) (**Figure 10B**). In the second case (Gould's model and C4c/CYFRA/CRP), the LR chi2 was 86.91 (p<0.001). The area under the ROC curve was 0.92 (95% CI=0.87-0.96) (**Figure 10C**). Other diagnostic characteristics of the two models are shown in **Table 19.**

**Table 19. Performance of lung cancer diagnostic models based on the plasma levels of molecular markers (C4c alone, or together with CYFRA 21-1 and CRP) and variables from the clinically validated Gould's model (age, smoking history, nodule diameter and time since quitting smoking).**

| | Gould's model + C4c | Gould's model + C4c/CYFRA 21-1/CRP |
|---|---|---|
| Sensitivity | 92% | 92% |
| Specificity | 66% | 72% |
| Positive predictive value¹ | 10% | 12% |
| Negative predictive value¹ | 99.5% | 99.6% |
| Likelihood positive ratio | 2.67 | 3.34 |
| Likelihood negative ratio | 0.12 | 0.11 |
| Correctly classified | 81% | 84% |

| | | |
|---|---|---|
| ¹Positive and negative predictive values were calculated with an estimated prevalence of malignant nodules in this clinical setting of 4%. | | |

### Conclusion

These analyses evidence the diagnostic capacity of the quantification of C4c in plasma samples (alone or in combination with other molecular markers and epidemiological and clinical features) for discriminating which pulmonary nodules are malignant. The application of this multimodality approach predicts lung cancer more accurately and may be used for the identification of the subjects that need more active follow-up, which would improve the clinical management of lung nodules by reducing the number of unnecessary procedures.

## Claims

1. *In vitro* method for the diagnosis of lung cancer in a subject which comprises:
a. Determining the level of at least C4c fragment in a plasma sample isolated from the subject; and
b. Comparing the C4c fragment level determined in step (a) with a reference control level of said C4c fragment, and
c. Wherein if the C4c fragment level determined in step (a) is higher than the reference control level, it is indicative that the subject suffers from lung cancer.

2. *In vitro* method, according to claim 1, wherein indeterminate pulmonary nodules have been previously identified in the subject to be diagnosed and wherein if the C4c fragment level determined in step (a) is higher than the reference control level, it is indicative that the indeterminate pulmonary nodules identified in the subject are malignant.

3. *In vitro* method for deciding whether to administer a medical treatment to a subject suspected of suffering from lung cancer which comprises:
a. Determining the level of at least C4c fragment in a plasma sample isolated from the subject; and
b. Comparing the C4c fragment level determined in step (a) with a reference control level of said C4c fragment, and
c. Wherein if the C4c fragment level determined in step (a) is higher than the reference control level, a medical treatment suitable for the treatment of lung cancer is selected to be administered to the patient.

4. *In vitro* method, according to claim 3, wherein indeterminate pulmonary nodules have been previously identified in the subject and wherein if the C4c fragment level determined in step (a) is higher than the reference control level, it is indicative that the pulmonary nodules identified in the subject are malignant and a medical treatment suitable for the treatment of lung cancer is selected to be administered to the patient.

5. *In vitro* method for determining the efficacy of a medical regimen in a patient already diagnosed with lung cancer which comprises:
a. Measuring the level of at least C4c fragment in a plasma sample isolated from the patient prior to the administration of the medical regimen; and
b. Measuring the level of said C4c fragment in a sample from the patient once started the administration of the medical regimen; and
c. Comparing the C4c fragment levels measured in steps (a) and (b), in such a way that if the C4c fragment level measured in step (b) is lower than the C4c fragment level measured in step (a), it is indicative that the medical regimen is effective in the treatment of lung cancer.

6. *In vitro* method, according to claim 5, wherein malignant pulmonary nodules have been previously identified in the patient and wherein if the C4c fragment level measured in step (b) is lower than the C4c fragment level measured in step (a), it is indicative that the medical regimen is effective in the treatment of malignant pulmonary nodules.

7. *In vitro* method for determining the efficacy of a medical regimen in a patient already diagnosed with lung cancer comprising:
a. Measuring the level of at least C4c fragment in a plasma from the patient once started the administration of the medical regimen; and
b. Comparing the C4c fragment level measured in step (i) with a reference control level of the C4c fragment, and
c. Wherein, if the C4c fragment level measured in step a) is not higher than the reference control level, it is indicative that the medical regimen is effective in the treatment of lung cancer.

8. *In vitro* method, according to claim 7, wherein malignant pulmonary nodules have been previously identified in the patient and wherein if the C4c fragment level measured in step a) is not higher than the reference control level, it is indicative that the medical regimen is effective in the treatment of malignant pulmonary nodules.

9. *In vitro* method, according to any of the previous claims, wherein the step a) comprises measuring the level of C4c fragment and prolactin, C4c fragment and CYFRA 21-1, C4c fragment and C-reactive protein (CRP), C4c fragment and prolactin and CYFRA 21-1, C4c fragment and prolactin and CRP, C4c fragment and CYFRA 21-1 and CRP, or C4c fragment and CYFRA 21-1 and CRP and prolactin.

10. *In vitro* method, according to any of the previous claims, wherein the lung cancer is selected from the group consisting of non-small cell lung cancer and small-cell lung carcinoma.

11. *In vitro* method, according to any of the previous claims, **characterized in that** it is an immunoassay.

12. Use of at least C4c fragment in the *in vitro* diagnosis of lung cancer.

13. Use, according to claim 12, of at least C4c fragment for the *in vitro* diagnosis of lung cancer, wherein it is determined if previously identified indeterminate pulmonary nodules are malignant.

14. Use, according to any of the claims 12 or 13, wherein the following combinations of biomarkers are measured: C4c and prolactin, C4c and CYFRA 21-1, C4c and CRP, C4c and prolactin and CYFRA 21-1, C4c and prolactin and CRP, C4c and CYFRA 21-1 and CRP, or C4c and CYFRA 21-1 and CRP and prolactin.
